# EUROPEAN PATENT APPLICATION

(11) **EP 2 957 320 A1**
(43) Date of publication of application: **23.12.2015**
(21) Application number: 14751857.5
(22) Date of filing: 14.02.2014
(51) Int. Cl.: A61N 1/32, H04R 1/00

(54) **LOW-FREQUENCY IMPOSITION DEVICE**

(30) Priority: 14.02.2013 JP 2013026631
(71) Applicant: YA-MAN LTD, Koto-ku, Tokyo 135-0045 (JP)
(72) Inventor: YAMAZAKI, Iwao, Tokyo 135-0045 (JP)
(74) Representative: Maury, Richard Philip
(86) International application number: PCT/JP2014/000770
(87) International publication number: WO 2014/125833

(57) **Abstract**

There is provided a low-frequency application device capable of applying a low-frequency voltage in synchronization with emitted music and preventing an unexpected large voltage from being suddenly supplied to a human body. A low-frequency application device, comprising: an audio signal supply unit to output audio signals; a filter unit to pass audio signals in a predetermined frequency band among the audio signals; a signal generation unit to generate a pulse signal by receiving an audio signal at a preset level or higher among the audio signals passed through the filter unit; electrodes to supply current to a human body; a low-frequency voltage generation unit to apply a generated pulsed low-frequency voltage to supply current corresponding to the voltage to the electrodes; and a control unit to control output of the voltage by the pulse signal.

## Description

### FIELD

Embodiments described herein relate generally to a low-frequency application device.

### BACKGROUND

Conventionally, there is a known low-frequency application device which can perform treatments such as fatigue recovery and muscle strengthening by bringing a pair of electrodes into contact with an arbitrary region of a human body and passing pulsed current between the electrodes to thereby cause contraction motion of the muscle so as to promote the flow of lymph. Causing the muscle to contract with current is referred to as EMS (Electrical Muscle Stimulation).
In such the low-frequency application device, various proposals have been made for the method of supplying current. One of the proposals is that the strength of sound is extracted from an inputted audio signal, and a voltage with a strength corresponding to music emitted from a speaker is supplied to a human body to pass current through the muscle (see, for example, Patent Reference 1).

### RELEVANT REFERENCES

### Patent References

Patent Reference 1: JP-A 2008-017979

### SUMMARY

### PROBLEM TO BE SOLVED BY THE INVENTION

However, in the low-frequency application devices, when a large noise is superposed on the inputted audio signal or when a music piece with a sudden loud sound is reproduced, an unexpected large voltage is sometimes suddenly supplied. The present invention has been made in consideration of the above circumstances, and its object is to provide a low-frequency application device capable of applying a low-frequency voltage in synchronization with emitted music and preventing an unexpected large voltage from being suddenly supplied to a human body.

### MEANS FOR SOLVING THE PROBLEMS

A low-frequency application device in an embodiment, includes: an audio signal supply means that outputs audio signals; a filter means that passes audio signals in a predetermined frequency band among the audio signals; a signal generation means that generates a pulse signal by receiving an audio signal at a preset level or higher among the audio signals passed through the filter means; electrodes for supplying current to a human body; a low-frequency voltage generation means that applies a generated pulsed low-frequency voltage to supply current corresponding thereto to the electrodes; and a control means that controls output of the low-frequency voltage by the pulse signal.

The low-frequency application device in an embodiment can also include a speaker that emits audio. The low-frequency application device in the present invention may further include a comparison means that compares a level of the audio signal passed through the filter means with a threshold value. Further, the low-frequency application device can further include a pulse width change means that changes a pulse width of the low-frequency voltage correspondingly to the threshold value.
It is desirable that in the low-frequency application device in the present invention, the filter means includes a first filter means that passes a low-frequency area of an audio signal for a right channel and a second filter means that passes a low-frequency area of an audio signal for a left channel. The low-frequency application device in the embodiment can further include a pass frequency change means that changes a frequency that the filter means passes.

The low-frequency application device in an embodiment desirably further include: first to third electrodes and first to third signal generation means; and an output electrode switch means that changeably supplies pulse signals outputted from the first to third signal generation means, to the first to third electrodes respectively. It is desirable that the first electrode corresponds to an audio signal for a right channel, and the second electrode corresponds to an audio signal for a left channel.
The low-frequency application device in an embodiment can further include a display means that displays at least one of information indicating an output mode of the low-frequency signal, presence or absence of output of the audio signal, a quality of audio emitted from the speaker, and information corresponding to change of the audio signal.

### EFFECT OF THE INVENTION

According to the low-frequency application device in the present invention, it becomes possible to apply a low-frequency voltage in synchronization with emitted music and prevent an unexpected large voltage from being suddenly supplied to a human body.

### BRIEF DESCRIPTION OF THE DRAWINGS

Fig. 1 is a block diagram illustrating an internal configuration of a low-frequency application device 1.
Fig. 2 is a flowchart illustrating an operation of a first A/D converter 132A.
Fig. 3 is a waveform chart illustrating audio signals after passing through a first filter circuit 131A.
Fig. 4 is a waveform chart illustrating trigger signals.
Fig. 5 is a chart illustrating trigger signals outputted from first to third A/D converters 132A to 132C.
Fig. 6 is a block diagram illustrating an internal configuration of a low-frequency application device 21 in a second embodiment.
Fig. 7 is a waveform chart explaining an operation of a first A/D converter 232A in the second embodiment.
Fig. 8 is a chart illustrating cycles of a low-frequency voltage decided by a pulse width setting circuit 280 in the second embodiment.

### DETAILED DESCRIPTION

### [First Embodiment]

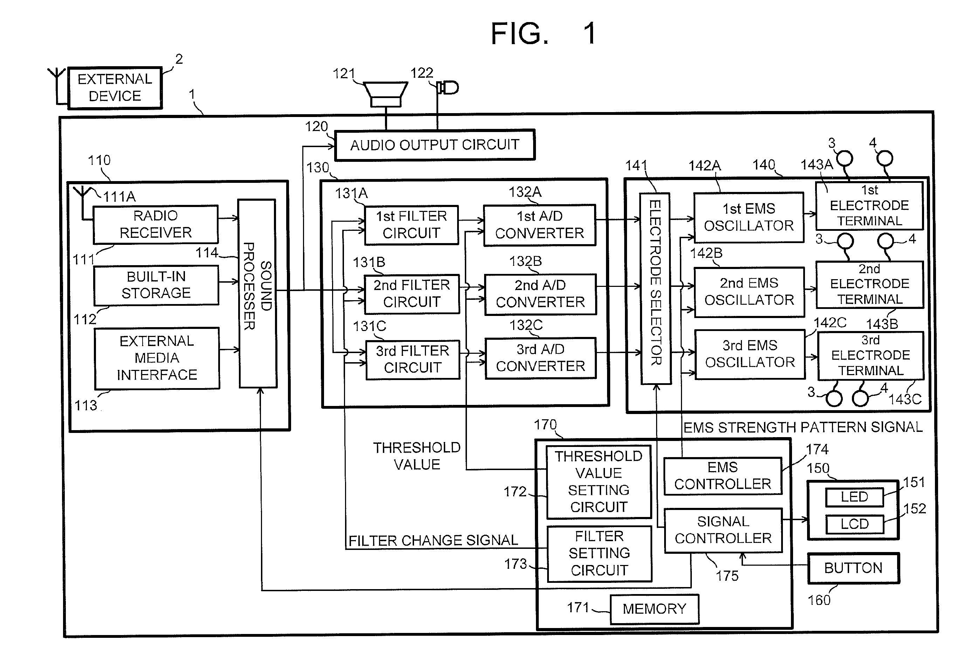

Hereinafter, an embodiment will be described based on the drawings. Fig. 1 is a block diagram illustrating an internal configuration of a low-frequency application device 1 in a first embodiment.
As illustrated in Fig. 1, the low-frequency application device 1 includes a sound source input circuit 110, an audio output circuit 120, a trigger signal generation circuit 130, an EMS output circuit 140, a display 150, a button 160, and a controller 170. The low-frequency application device 1 may have a DC power supply for supplying power to respective parts, for example the controller 170. The DC power supply includes a secondary battery or a rectifying circuit for performing AC-DC conversion on the commercial power supply obtained from a receptacle at home.

The low-frequency application device 1 increases an output voltage of the built-in DC power supply up to about 100 V at most. The low-frequency application device 1 can apply a low to middle-frequency voltage (hereinafter, referred to as a "low-frequency voltage") of about 10 kHz or less to a skin surface of a person to be treated via detachable electrode pads 3, 4. In this event, the low-frequency application device 1 can synchronize ON/OFF of the low-frequency voltage to be applied, with music outputted from a speaker 121 and an earphone 122.
Applying the low-frequency voltage to the person to be treated makes it possible to promote the flow of lymph of the person to be treated. It is also possible to obtain treatment effects such as fatigue recovery and so on. Furthermore, applying the low-frequency voltage to the person to be treated in synchronization with music makes it possible for the person to be treated to receive the treatments such as promotion of the flow of lymph and fatigue recovery and muscle-building exercise by electrical muscle stimulation with enjoyment. This makes it possible for a user to be more likely to continuously use the low-frequency application device 1. The low-frequency application device 1 desirably has a water proofing property to be usable also in a both room.

### (Regarding the sound source input circuit 110)

The sound source input circuit 110 includes a radio receiver 111, a built-in storage 112, an external media interface 113, and a sound processer 114. The radio receiver 111 receives music files in various formats such as an AAC (Advanced Audio Coding) format and MP3 (MPEG Audio Layer-3) format and audio signals after decoding such music files, by radio communication from an external device 2 via an antenna 111A. Note that the "audio signals" include a signal containing only human voice, a signal containing both human voice and music, and a signal containing only music (so-called instrumental). The radio receiver 111 is compatible with radio communication in various standards such as Bluetooth (registered trademark), Wi-Fi (registered trademark), wireless LAN, infrared ray and so on. The radio receiver 111 can also receive radio audio signals transmitted from broadcast stations.

The built-in storage 112 is a flash memory capable of holding music files.
Into the external media interface 113, music files and audio signals are inputted from other than the external device 2.
The external media interface 113 is provided with not-illustrated wired LAN port, line input terminal, USB (Universal Serial Bus) terminal, SD card insertion port. To the external media interface 113, a microphone for picking sound around the low-frequency application device 1 is attached in some cases.
The sound processer 114 is an audio signal supply means that outputs audio signals to the audio output circuit 120 or the trigger signal generation circuit 130.
The sound processer 114 has a function of selecting an output source of a music file and an audio signal, a function of bypassing an audio signal, a function of decoding an audio signal or encoding a music file, and a function of separating an audio signal to a right channel or a left channel.

For example, the sound processer 114 selects and decides an output source of the music file or the audio signal from among the radio receiver 111, the built-in storage 112, and the external media interface 113.
For example, the sound processer 114 reads and decodes a music file in the MP3 format from the built-in storage 112 and outputs audio signals to the audio output circuit 120 and the trigger signal generation circuit 130.

When a decoded audio signal transmitted from the external device 2 is inputted into the sound processer 114 via the radio receiver 111, the sound processer 114 outputs the decoded audio signal, as it is without encoding it, to the audio output circuit 120 and the trigger signal generation circuit 130.
There is a case where the audio signal is a stereo signal. The sound processer 114 can output the stereo signal as it is to the audio output circuit 120 and the trigger signal generation circuit 130.
Further, the sound processer 114 can also separate an audio signal for the right channel and an audio signal for the left channel contained in the stereo signal, and output them independently to the audio output circuit 120 and the trigger signal generation circuit 130.
There is a case where the audio signal is a monaural signal. In this case, the sound processer 114 outputs the audio signal as the monaural signal to the audio output circuit 120 and the trigger signal generation circuit 130.

### (Regarding the audio output circuit 120)

The audio output circuit 120 is a connection part for detachably connecting the speaker 121 and the earphone 122 to the low-frequency application device 1. The speaker 121 is not detachably connected to but may be built in the low-frequency application device 1.
The audio output circuit 120 includes a not-illustrated amplifier circuit and thus amplifies the audio signal outputted from the sound processer 114 and outputs the amplified audio signal to the speaker 121 and the earphone 122.

### (Regarding the trigger signal generation circuit 130)

The trigger signal generation circuit 130 generates a pulsed trigger signal for synchronizing timings of audio emitted from the speaker 121 and the earphone 122 and application of a pulse voltage to the person to be treated.

The trigger signal generation circuit 130 includes first to third filter circuits 131A to 131C and first to third A/D converters 132A to 132C.

### (Regarding the first to third filter circuits 131A to 131C)

The first to third filter circuits 131A to 131C are band-pass filters each realized by using, for example, an operational amplifier. The first to third filter circuits 131A to 131C pass audio signals in a predetermined frequency band (for example, a band in which a low range and a melody are included) among audio signals outputted from the sound processer 114. In the case of passing the band in which a melody is included among the audio signals, it is desirable to set the cut-off frequency so that a pass band becomes about 100 Hz to about 2 kHz.

The first to third filter circuits 131A to 131C may be low-pass filters. In this case, the first to third filter circuits 131A to 131C can be constituted by using quintic Butterworth filters or Sallen-key filters. The first to third filter circuits 131A to 131C in this case are ideally have a cut-off frequency of about 100 Hz as a frequency characteristic. Further, the first to third filter circuits 131A to 131C desirably have a gain of -3dB near 150 Hz and a gain of -60 dB near 200 Hz. The first to third filter circuits 131A to 131C desirably permit ripple and have sharp cut-off characteristics.
In the following description, it is assumed that the audio signals for the right channel among the audio signals are inputted from the sound processer 114 into the first filter circuit 131A. The first filter circuit 131A passes the audio signals in a low-frequency area for the right channel.

In the following description, it is assumed that the audio signals for the left channel among the audio signals are inputted from the sound processer 114 into the second filter circuit 131B. The second filter circuit 131B passes the audio signals in a low-frequency area for the left channel.
In the following description, the audio signals as the stereo signals are inputted from the sound processer 114 into the third filter circuit 131C. The third filter circuit 131C is assumed to pass the audio signals in a middle to high-frequency area corresponding to the melody.
The frequency band which each of the first to third filter circuits 131A to 131C passes can be changed, as necessary, in a range of 50 Hz to 2 kHz.

### (Regarding the first to third A/D converters 132A to 132C)

The first to third A/D converters 132A to 132C are signal generators that generate trigger signals using the audio signals at predetermined levels or higher among the audio signals passed through the first to third filter circuits 131A to 131C.
The first to third A/D converters 132A to 132C hold threshold values required when generating the trigger signals in the not-illustrated memory.
The threshold values can be changed as necessary depending on the category of music to which a music signal corresponds. For example, operating the button 160 by a treating person makes it possible to change the threshold value. The "music category" is, for example, ROCK, POPS, JAZZ or CLASSIC.

Into the first A/D converter 132A, the audio signals passed through the first filter circuit 131A are inputted. Similarly, into the second and third A/D converters 132B, 132C, the audio signals passed through the second and third filter circuits 131B, 131C are inputted respectively.

### (Concrete example of generation of the trigger signal by the first A/D converter 132A)

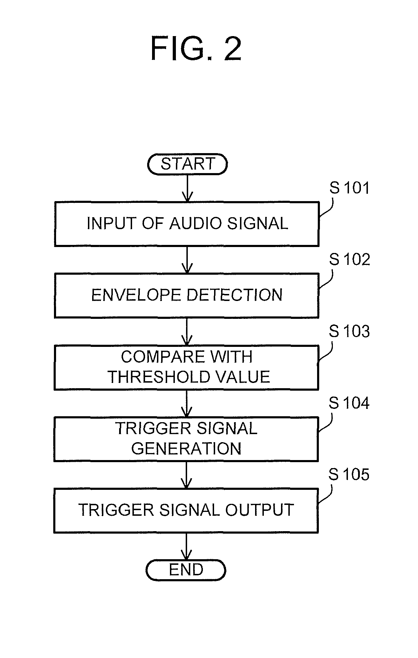

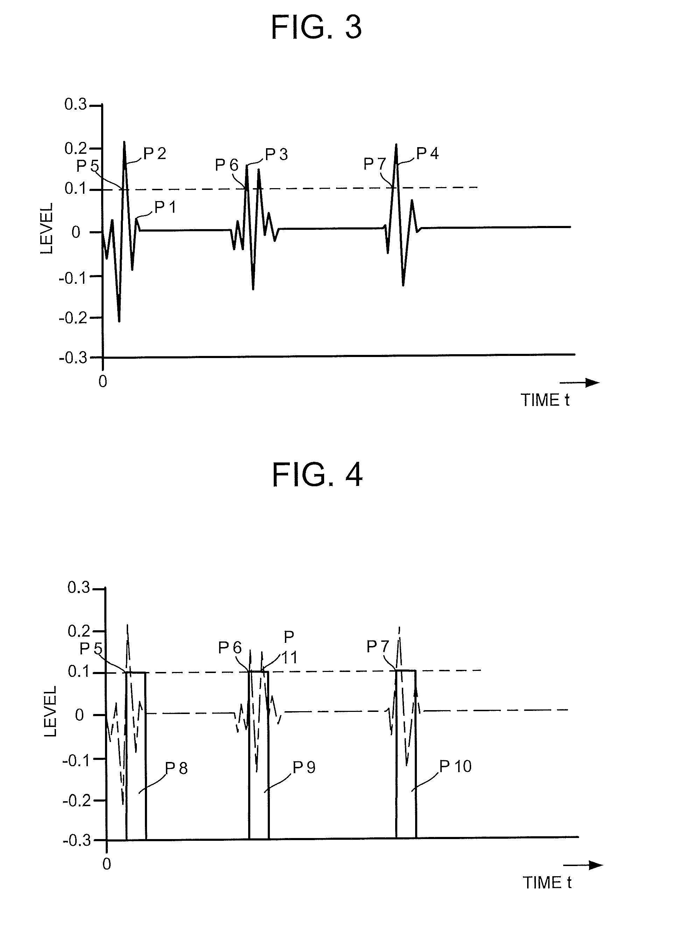

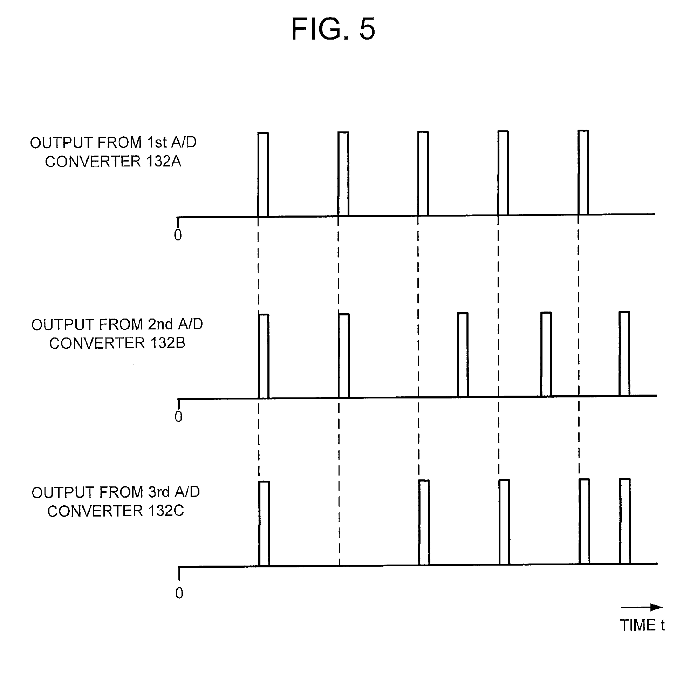

The first to third A/D converters 132A to 132C generate pulsed trigger signals with predetermined widths on the basis of the audio signals inputted thereinto.
Here, the generation of the trigger signal by the first A/D converter 132A will be described in detail referring to Fig. 2 to Fig. 5. Fig. 2 is a flowchart illustrating the operation of the first A/D converter 132A. Fig. 3 is a waveform chart illustrating the audio signals after passing through the first filter circuit 131A. Fig. 4 is a waveform chart illustrating the trigger signals. Fig. 5 is a chart illustrating the trigger signals outputted from the first to third A/D converters 132A to 132C.

Note that the second and third A/D converters 132B, 132C have the same functions as that of the first A/D converter 132A, and therefore detailed description of the second and third A/D converters 132B, 132C will be omitted.

### (1) Input of audio signal (Step S101)

Into the first A/D converter 132A, the audio signals passed through the first filter circuit 131A are inputted (see Fig. 3). Note that the first filter circuit 131A is configured to pass only the audio signals of 50 Hz or less here as an example.
Therefore, it is assumed that into the first A/D converter 132A, audio signals corresponding to the sounds of musical instruments (for example, a bass and a bass drum) in a low range among the audio signals outputted from the sound processer 114 are inputted.

### (2) Envelope detection (Step S102)

The first A/D converter 132A performs envelope detection on the audio signals inputted thereinto. As a result, a waveform at a small amplitude level among the audio signals (see, for example, numeral P1 in Fig. 3) is smoothed, so that only the audio signals at a certain level or higher (see, for example, numerals P2 to P4 in Fig. 3) are detected.
The first A/D converter 132A can detect components from DC to 2 kHz included in the audio signals.

### (3) Pulse shape generation processing (Steps S103, S104)

The first A/D converter 132A compares the level of the audio signal subjected to the envelope detection with the held threshold value to extract an audio signal at the same level as the threshold value (see a broken line in Fig. 3) (Step S103). Here, it is assumed that the threshold value is, as an example, a value with which an audio signal of -20 dB or higher among the levels of the entire audio signals can be extracted.

As illustrated in Fig. 4, the first A/D converter 132A generates trigger signals (see numerals P8 to P10 in Fig. 4) using intersections of the audio signals and the threshold value (see numerals P5 to P7 in Fig. 3 and Fig. 4) as rising edges (Step S104). The pulse width of the trigger signal is decided by a flip-flop circuit.
The trigger signals thus generated correspond to timings of output of the sounds that are easily perceived as sounds or vibrations by the person to be treated, among the sounds outputted from the speaker 121 and the earphone 122.
Note that when the audio signals at the threshold value or higher are sometimes successive in a short time (see numerals P6 and P11 in Fig. 4). In this case, it is desirable to generate no trigger signal for the temporary subsequent audio signal (see numeral P11). In this case, the trigger signals are not successively outputted in a short time, resulting in application of no low-frequency voltage in a short time to the person to be treated. This can prevent slight pinprick-like pain or discomfort feeling such as pins and needles from being given to the person to be treated.

### (4) Trigger signal output (Step S105)

The first A/D converter 132A outputs the trigger signals generated as described above to the EMS output circuit 140. The second and third A/D converters 132B, 132C also similarly generate trigger signals and output them to the EMS output circuit 140. As a result, the trigger signals as illustrated in Fig. 5 are outputted to the EMS output circuit 140 from the first to third A/D converters 132A to 132C.

### (Regarding the EMS output circuit 140)

The EMS output circuit 140 will be described referring to Fig. 1 again. The EMS output circuit 140 includes an electrode selector 141, first to third EMS oscillators 142A to 142C, and first to third electrode terminals 143A to 143C.

The electrode selector 141 is an output electrode switch that changeably supplies the trigger signals which are inputted thereinto from the first to third A/D converters 132A to 132C, to the first to third EMS oscillators 142A to 142C. For example, the electrode selector 141 can output the trigger signals which are inputted thereinto from the first A/D converter 132A, to the third EMS oscillator 142C. For example, the electrode selector 141 can output the trigger signals which are inputted thereinto from the third A/D converter 132C, to the second EMS oscillator 142B.
The first to third EMS oscillators 142A to 142C can generate pulsed low-frequency voltages. The first to third EMS oscillators 142A to 142C are voltage generators that apply the pulsed low-frequency voltages to the first to third electrode terminals 143A to 143C to supply currents corresponding thereto to the person to be treated via the electrode pads 3, 4. Concretely, the first to third EMS oscillators 142A to 142C apply the low-frequency voltages to the first to third electrode terminals 143A to 143C in synchronization with the trigger signals inputted thereinto. As a result, the low-frequency voltages are applied to the first to third electrode terminals 143A to 143C in synchronization with the audio signals outputted from the sound processer 114 to the audio output circuit 120.
The first to third EMS oscillators 142A to 142C desirably have excellent response to the trigger signals, and desirably employ transformer type circuits as their output circuits for outputting the low-frequency voltages.

The first to third electrode terminals 143A to 143C are electrode parts that apply the generated pulsed low-frequency voltages to a human body to supply currents corresponding thereto to the skin.
To each of the first to third electrode terminals 143A to 143C, pair of electrode pads 3, 4 is connected. The electrode pads 3, 4 are pasted to the skin surface of the person to be treated. As a result of this, the low-frequency voltages from the first to third electrode terminals 143A to 143C are applied to the skin of the person to be treated and low-frequency currents flow through the muscle of the person to be treated.
The low-frequency currents are transmitted to the body and a specific part of the body of the person to be treated, so that the person to be treated can experience EMS in synchronization with the music outputted from the speaker 121 and the earphone 122.

Here, it is assumed that a low-frequency voltage corresponding to the audio signal for the right channel is applied to the first electrode terminal 143A. It is assumed that a low-frequency voltage corresponding to the audio signal for the left channel is applied to the second electrode terminal 143B. It is assumed that a low-frequency voltage corresponding to the melody is applied to the third electrode terminal 143C.

### (display 150)

The display 150 includes an LED (Light Emitting Diode) 151 and an LCD (Liquid Crystal Display) 152. The LED 151 and the LCD 152 are report unit that report at least one of pieces of information indicating the presence or absence of the low-frequency voltages generated by the first to third EMS oscillators 142A to 142C and the mode (the frequencies and strengths of the low-frequency voltages to be generated), the presence or absence of the audio signals outputted from the sound processer 114 to the audio output circuit 120 or the trigger signal generation circuit 130, and the quality and volume of the audio emitted from the speaker 121 and the earphone 122.

For example, in the case where the audio signals are being outputted from the sound processer 114 to the audio output circuit 120 or the trigger signal generation circuit 130, the LED 151 lights up.
The LED 151 can be made to blink to be synchronous with the audio signals outputted to the audio output circuit 120 or the trigger signal generation circuit 130. In this case, the LED 151 blinks to be synchronous with the music emitted from the speaker 121 and the earphone 122. In the case of making the LED 151 blink as described above, it is only necessary to output the trigger signals generated by the first to third A/D converters 132A to 132C, to a signal controller 175.
It is also possible to make the LCD 152 display graphics (indicator in a bar shape and a screen saver moving up and down in concert with the sound timing) synchronizing with the audio signals outputted to the audio output circuit 120 or the trigger signal generation circuit 130. In this case, the graphics are displayed on the LCD 152 to synchronize with the music outputted from the speaker 121 and the earphone 122. As described above, making the LED 151 brink or the LCD 152 display the graphics leads to expectation of the effects that the person to be treated finds EMS by the low-frequency voltage more pleasurable.
The display 150 may be configured to include only either the LED 151 or the LCD 152.

### (Regarding the button 160)

The button 160 is a physical switch that accepts an operation by the treating person. An example of the "operation" is selection of from which of the radio receiver 111, the built-in storage 112, and the external media interface 113 the audio signals are obtained.
Another example of the "operation" is change of the frequency bands which the first to third filter circuits 131A to 131C pass. Other examples of the "operation" include an operation of changing the threshold values held in the first to third A/D converters 132A to 132C, change of the strengths, the frequencies and the application patterns of the low-frequency voltages applied by the first to third EMS oscillators 142A to 142C and so on. Note that it is also possible to make the LCD 152 display an image corresponding to the button 160 to eliminate provision of the physical button 160.

### (Regarding the controller 170)

The controller 170 includes a memory 171, a threshold value setting circuit 172, a filter setting circuit 173, an EMS controller 174, and the signal controller 175 that controls them.
The memory 171 stores, in advance, a plurality of pieces of pass frequency band information indicating the frequency bands which the first to third filter circuits 131A to 131C pass and a plurality of threshold values held in the first to third A/D converters 132A to 132C.
The memory 171 stores, in advance, a plurality of pieces of low-frequency level information indicating the strengths of the low-frequency voltages generated by the first to third EMS oscillators 142A to 142C.
The memory 171 stores, in advance, information indicating the lighting pattern of the LED 151 and information which the LCD 152 is made to display. The memory 171 stores, in advance, information indicating a plurality of waveform patterns of the low-frequency voltages. It is possible to give different kinds of stimulation to the person to be treated (for example, "pricking" stimulation like tapping and "creeping" stimulation like being pressed with a someone's finger) according to the "plurality of waveform patterns."

The threshold value setting circuit 172 is a threshold value change unit that changes the threshold values held in the first to third A/D converters 132A to 132C. The threshold value setting circuit 172 reads the threshold values from the memory 171 and outputs them to the first to third A/D converters 132A to 132C to cause the first to third A/D converters 132A to 132C to write the read threshold values over the threshold values held in the first to third A/D converters 132A to 132C.
The filter setting circuit 173 is a pass frequency changer that changes the pass frequencies of the audio signals of the first to third filter circuits 131A to 131C. The filter setting circuit 173 reads the pass frequency band information from the memory 171 and outputs filter change signals corresponding to the pass frequency band information to the first to third filter circuits 131A to 131C. As a result of this, the frequency bands which the first to third filter circuits 131A to 131C pass are changed.

The EMS controller 174 supplies power to the first to third EMS oscillators 142A to 142C. In this event, the EMS controller 174 reads the pieces of low-frequency level information from the memory 171. The EMS controller 174 then supplies power corresponding to the pieces of low-frequency level information to the first to third EMS oscillators 142A to 142C. This makes it possible to easily change the strengths of the low-frequency voltages to be applied to the first to third electrode terminals 143A to 143C.
The EMS controller 174 can also output the information indicating the waveform patterns of the low-frequency voltages to be applied to the first to third electrode terminals 143A to 143C, to the first to third EMS oscillators 142A to 142C. As a result of this, various patterns of EMS can be given to the person to be treated.

The signal controller 175 can detect the operation of the button 160 by the treating person. The signal controller 175 controls the threshold value setting circuit 172, the filter setting circuit 173, the EMS controller 174 or the sound processer 114 according to the detected operation. For example, the signal controller 175 controls the sound processer 114 to select and decide which of the radio receiver 111, the built-in storage 112, and the external media interface 113 is used as the output source of the audio signal. According to the low-frequency application device 1 configured as described above, pasting the electrode pads 3, 4 connected to the first to third electrode terminals 143A to 143C to the body or a specific part of the body of the person to be treated makes it possible to apply low-frequency voltages in synchronization with the audio signals outputted from the sound processer 114 to the skin of the person to be treated.

For example, by pasting the electrode pads 3,4 connected to the first electrode terminal 143A and the second electrode terminal 143B, to which low-frequency voltages in synchronization with the sounds in the low range are applied, to a region of the body with a large muscle mass (abdominal muscle, thigh muscle, or back muscle), the person to be treated becomes easy to get a sense of presence as if listening to music in a live concert hall.
Further, by pasting the electrode pads 3, 4 connected to the third electrode terminal 143C to, for example, a shoulder of the person to be treated, the person to be treated can feel the low-frequency stimulation in synchronization with the sound of the melody at the shoulder.
In this event, according to the low-frequency application device 1, only the pulse signals corresponding to the rhythm of the music are generated from the audio signals outputted from the sound processer 114, and the strengths of the low-frequency voltages are controlled by the EMS controller 174.
This can prevent an unexpected large current from being suddenly supplied. In short, according to the low-frequency application device 1, it is possible to apply the low-frequency voltage to the skin of the user in synchronization with the music signal outputted from the sound processer 114 and to prevent an unexpected large voltage from being suddenly supplied to the human body.

### [second embodiment]

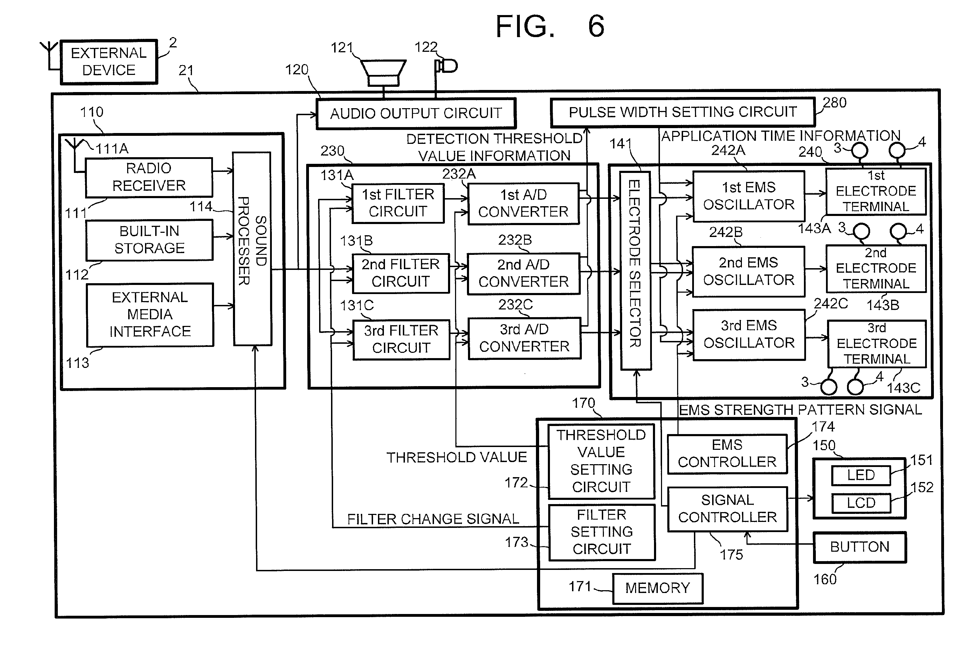

Next, a second embodiment will be described based on Fig. 6 to Fig. 8. Fig. 6 is a block diagram illustrating an internal configuration of a low-frequency application device 21 in the second embodiment.

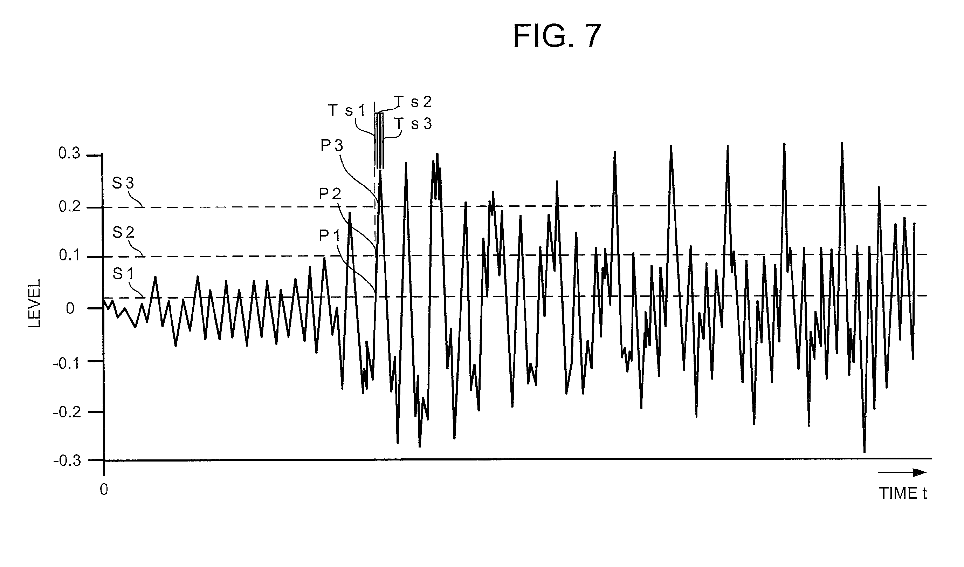

Fig. 7 is a waveform chart explaining an operation of a first A/D converter 232A in the second embodiment.

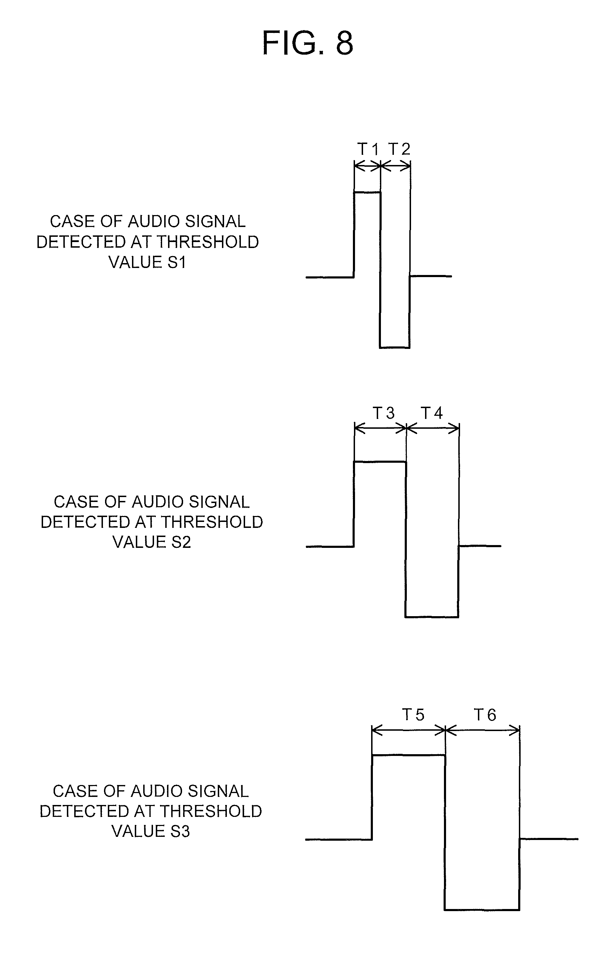

Fig. 8 is a chart illustrating cycles of a low-frequency voltage decided by a pulse width setting circuit 280 in the second embodiment. Note that in Fig. 6, the same components as those of the low-frequency application device 1 in the first embodiment illustrated in Fig. 1 to Fig. 5 are given the same reference numerals, and their description will be omitted.
The low-frequency application device 21 in the second embodiment includes, as illustrated in Fig. 6, a trigger signal generation circuit 230 and an EMS output circuit 240 in place of the trigger signal generation circuit 130 and the EMS output circuit 140 included in the low-frequency application device 1 in the first embodiment. The low-frequency application device 21 further includes the pulse width setting circuit 280.

The trigger signal generation circuit 230 includes first to third A/D converters 232A to 232C corresponding to the first to third A/D converters 132A to 132C included in the low-frequency application device 1 in the first embodiment. The first to third A/D converters 232A to 232C can omit the envelope processing for reduction of signal processing time.

### (Regarding the first A/D converter 232A)

Hereinafter, the first A/D converter 232A will be described. The second and third A/D converters 232B, 232C have the same configurations and functions as those of the first A/D converter 232A, and therefore detailed description of the second and third A/D converters 232B, 232C will be omitted.

As illustrated in Fig. 7, the first A/D converter 232A holds a plurality of threshold values S1 to S3 and compares an audio signal inputted thereinto with the held threshold values S1 to S3 at the same time. Upon detection of an audio signal of the threshold value S1 or higher, the first A/D converter 232A generates a trigger signal. Then, the first A/D converter 232A outputs the trigger signal to the electrode selector 141. For example, there is a case where audio signals of threshold values S2 and S3 or higher are sometimes detected immediately after the detection of the audio signal of the threshold value S1 or higher (see numerals P1 to P3 in Fig. 7). In this case, three trigger signals are successively generated in a short time (see Ts1 to Ts3 in Fig. 7).
The first A/D converter 232A outputs detection threshold value information indicating at which threshold value among the threshold values S1 to S3 the first A/D converter 232A has detected the audio signal, to the pulse width setting circuit 280 at the same time with the generation of the trigger signal.

### (Regarding the EMS output circuit 240)

As illustrated in Fig. 6, the EMS output circuit 240 includes first to third EMS oscillators 242A to 242C corresponding to the first to third EMS oscillators 142A to 142C included in the low-frequency application device 1 in the first embodiment. The first to third EMS oscillators 242A to 242C receive input of trigger signals from the electrode selector 141 respectively.
The EMS output circuit 240 receives application time information indicating application time per cycle of the low-frequency voltage from the pulse width setting circuit 280.
The first to third EMS oscillators 242A to 242C are supplied with power from the EMS controller 174, and apply the low-frequency voltages with cycles corresponding to the received application time information to the first to third electrode terminals 143A to 143C.

### (Regarding the pulse width setting circuit 280)

The pulse width setting circuit 280 is a pulse width changer that changes the pulse widths of the low-frequency voltages to be applied by the first to third EMS oscillators 242A to 242C.
The pulse width setting circuit 280 decides the pulse widths of the low-frequency voltages correspondingly to the detection threshold value information outputted from the first to third A/D converters 232A to 232C as illustrated in Fig. 8.
The pulse width is decided according to the application time for one cycle of the low-frequency voltage. Concretely, when the detection threshold value information received from the first to third A/D converters 232A to 232C indicates the threshold value S1, the pulse width setting circuit 280 decides application of a low-frequency voltage of one cycle of 40 µsec with each application time T1, T2 of the low-frequency voltage set to 20 µsec.

When the detection threshold value information received from the first to third A/D converters 232A to 232C indicates the threshold value S2, the pulse width setting circuit 280 decides application of a low-frequency voltage of one cycle of 80 µsec with each application time T3, T4 of the low-frequency voltage set to 40 µsec. When the detection threshold value information received from the first to third A/D converters 232A to 232C indicates the threshold value S3, the pulse width setting circuit 280 decides application of a low-frequency voltage of one cycle of 160 µsec with each application time T5, T6 of the low-frequency voltage set to 80 µsec. The pulse width setting circuit 280 outputs the decided application time of one cycle of the low-frequency voltage as the application time information to each of the first to third EMS oscillators 242A to 242C.
The pulse width setting circuit 280, the first to third A/D converters 232A to 232C, and the first to third EMS oscillators 242A to 242C operate in synchronization with one another by a not-illustrated synchronization clock.

According to the low-frequency application device 21 configured as described above, the first A/D converter 232A holds the plurality of threshold values S1 to S3, and compares an audio signal inputted thereinto with the held threshold values S1 to S3 at the same time. Thus, the pulse signal is periodically outputted in a short period. As a result, a period when the low-frequency voltage is not outputted can be reduced. If the period when the low-frequency voltage is not outputted becomes long, the person to be treated maybe possibly bored. According to the low-frequency application device 21, such a situation can be prevented.
Further, according to the low-frequency application device 21, the pulse width setting circuit 280 can change the application time per cycle of the low-frequency voltage according to the detection threshold value information. For example, when the detection threshold value information corresponding to the threshold value S3 for detecting the audio signal at a high level is outputted, the pulse width setting circuit 280 sets the one cycle of the low-frequency voltage to a long time.

Further, when the detection threshold value information corresponding to the threshold value S1 for detecting the audio signal at a low level is outputted, the pulse width setting circuit 280 sets the one cycle of the low-frequency voltage to a short time. As a result of this, when the first to third A/D converter 232A to 232C detects an audio signal with a small peak, the pulse width of the low-frequency voltage becomes narrower. Accordingly, more frequent stimulation will be given to the person to be treated. When the first to third A/D converter 232A to 232C detects an audio signal with a large peak, the pulse width of the low-frequency voltage becomes wider. Accordingly, infrequent stimulation will be given to the person to be treated. In short, according to the low-frequency application device 21, the stimulation of the low-frequency voltage can be changed according to the magnitude of the level of the amplitude of the audio signal.

Further, in the low-frequency application device 21, the EMS controller 174 can control the strength of the low-frequency voltage, thereby making it possible to prevent an unexpected large current from being suddenly supplied.

### [Other Examples]

The first and second embodiments of the present invention have been described above, and the present invention is not limited to the above embodiments but can be variously changed. For example, the present invention may have a configuration without part of the above-described configuration (such as the third electrode terminal 143C).

## Claims

1. A low-frequency application device, comprising:
an audio signal supply unit to output audio signals;
a filter unit to pass audio signals in a predetermined frequency band among the audio signals;
a signal generation unit to generate a pulse signal by receiving an audio signal at a preset level or higher among the audio signals passed through the filter unit;
electrodes to supply current to a human body;
a low-frequency voltage generation unit to apply a generated pulsed low-frequency voltage to supply current corresponding to the voltage to the electrodes; and
a control unit to control output of the voltage by the pulse signal.

2. The low-frequency application device according to claim 1, further comprising a speaker to emit audio corresponding to the audio signal.

3. The low-frequency application device according to claim 1 or 2, further comprising a comparison unit to compare a level of the audio signal passed through the filter unit with a threshold value,
wherein the signal generation unit detects an audio signal at a level of the threshold value or higher.

4. The low-frequency application device according to claim 3, further comprising a pulse width change unit to change a pulse width of the voltage correspondingly to the threshold value.

5. The low-frequency application device according to any one of claims 1 to 4,
wherein the filter unit comprises a first filter to pass a low-frequency area of an audio signal for a right channel and a second filter to pass a low-frequency area of an audio signal for a left channel.

6. The low-frequency application device according to any one of claims 1 to 5, further comprising a pass frequency change unit to change a passband of the filter unit.

7. The low-frequency application device according to any one of claims 1 to 6, further comprising:
wherein the electrodes include a first to third pair of electrodes and the signal generation unit includes a first to third signal generator,
the low-frequency application device further comprises an output electrode switch to change combination of the first to third signal generation unit and the first to third pair of electrodes respectively.

8. The low-frequency application device according to claim 7,
wherein the first pair of electrodes corresponds to an audio signal for a right channel, and the second pair of electrodes corresponds to an audio signal for a left channel.

9. The low-frequency application device according to any one of claims 1 to 8, further comprising a display to display at least one of information indicating an output mode of the voltage, information indicating presence or absence of output of the audio signal, information indicating a quality of audio emitted from the speaker, and information corresponding to change of the audio signal.
